Europäisches Patentamt

(19) **European Patent Office**

**Office européen des brevets**

(11) Veröffentlichungsnummer: **0 138 178**
A2

# (12) EUROPÄISCHE PATENTANMELDUNG

(21) Anmeldenummer: **84112020.7**

(22) Anmeldetag: **06.10.84**

(51) Int. Cl.⁴: **A 61 L 15/00**

(30) Priorität: **14.10.83 DE 3337444**

(43) Veröffentlichungstag der Anmeldung: **24.04.85**
**Patentblatt 85/17**

(84) Benannte Vertragsstaaten: **AT BE CH DE FR GB IT LI LU NL SE**

(71) Anmelder: **Henkel Kommanditgesellschaft auf Aktien, Postfach 1100 Henkelstrasse 67, D-4000 Düsseldorf-Holthausen (DE)**

(72) Erfinder: **Sustmann, Scarlet, Dr., Am Nachtigallenwäldchen 34, D-4060 Viersen 12 (DE)**
Erfinder: **Marini, Ingo Gerhard, Dr., Hauptstrasse 40, A-4860 Lenzing (AT)**

(54) **Verwendung von den pH-Wert regulierenden Materialien für absorbierende Hygienemittel.**

(57) Die Erfindung betrifft die Verwendung von den pH-Wert regulierenden Materialien, wobei der sie bildende Stoff eine einheitliche, azide, chemisch modifizierte Viskosefaser ist, für verschiedene Hygienemittel.

Henkelstraße 67

4000 Düsseldorf, den 11.1o.1983

— 1 —

HENKEL KGaA

ZR-FE/Patente

AvK/Dr.JG

0138178

**Patentanmeldung**

**D 6861** EP

BEZEICHNUNG GEÄNDERT
siehe Titelseite

Die Verwendung von den pH-Wert regulierenden
Materialien

Die Erfindung betrifft die Verwendung von den pH-Wert
regulierenden Materialien, wobei der sie bildende
Stoff eine einheitliche azide, chemisch modifizierte
Faser ist.

Absorbierende Materialien, die für Hygienemittel, insbesondere Menstruationstampons, Binden o.ä. verwendet
werden, bestehen aus hydrophilen Faserstoffen, wie
Baumwoll-Lintern, Viskose, Schäumen o.ä.

Sofern die entsprechenden Hygienemittel in direkten
Hautkontakt kommen, hat es sich als vorteilhaft erwiesen, die zur Herstellung der Mittel verwendeten
Materialien mit Zusätzen zu versehen, die den pH-Wert
der sie umgebenden Haut positiv beeinflussen und dadurch Reizungen bzw. erhöhte Anfälligkeit gegenüber
Erkrankungen verhindern sollen.

So wurden z.B. Tampons vorgeschlagen (DT 309575), die
mit Glycogen, Zucker, Döderlein-Bakterien, Östrogenen
etc. imprägniert sind, u.a. also solchen Substanzen,
aus denen letztlich durch Lactobacillus acidophilus
Milchsäure gebildet wird. Diese Zusätze haben jedoch
den Nachteil, daß gerade während der Menstruation,
wenn der pH-Wert der Vagina im alkalischen Bereich
liegt, diese Bakterien in ihrem Wachstum stark gehemmt sind, so daß die Erzeugung von Milchsäure unter diesen Bedingungen nicht in wünschenswertem Ausmaß stattfinden kann. Außerdem wurde auch die unmittelbare Beaufschlagung der Materialien mit Milchsäure, Citronensäure u.ä. vorgeschlagen, DT 309575 und US-PS 979 499. Derartige Imprägnierungen bzw. Zuätze haben den weiteren Nachteil, daß aufgrund der hohen Pufferwirkung der Menstruationsflüssigkeit bei den mit einem Tampon anzubietenden geringen Säuremengen die Wirkung bereits bei geringem Blutanfall erschöpft ist.

Die DE-OS 32 36 768 beschreibt Tampons, die eine oder mehrere Substanzen enthalten, die während der Benutzung des Tampons einen pH-Wert zwischen 2,5 und 4,5 erzeugen und aufrechterhalten und damit das Wachstum von pathogenen Bakterien verhindern. Als derartige Substanzen werden monomere und/oder polymere physiologisch verträgliche Carbonsäuren, wie Citronensäure, Äpfelsäure, Weinsäure oder Milchsäure genannt. Nachteil derartiger Tampons ist ebenfalls, daß die hohe Pufferwirkung der Körperflüssigkeiten der acidifizierenden Wirkung der in den Tampon eingebrachten Säuren Grenzen setzt.

In der US-PS 3 187 747 werden absorbierende Faser-Materialien mit Ionen-Austausch-Eigenschaften beschrieben, deren Zusammensetzung durch ein Vielkomponentensystem von Polymeren gekennzeichnet ist: Polymerkomponenten mit faserbildenden Eigenschaften, beispielsweise Textilfasern, liegen neben anderen Polymeren mit acidifizierenden Eigenschaften, beispielsweise Carboxymethylcellulose, in Form sogenannter "Polymer-Legierungen" ("alloy") vor, die aus homogenen Lösungen geeigneter Polymere erhalten werden können. Die Heterogenität der Ausgangs-Polymeren bedingt, daß die Verarbeitung der Polymer-Legierungen zu den gewünsch-

Patentanmeldung    D6861 EP

HENKEL KGaA
ZR-FE/Patente

0138178

ten Hygienemitteln zusätzliche Verfahrensschritte, wie z.B. eine Lösungsmittel-Austauschtrocknung, erfordert, um ein Entquellen der Fasern zu bewirken und damit zu verhindern, daß sie während des Trocknungsvorganges aneinander kleben. Außerdem werden derartige Fasern häufig noch mit kationenaktiven Weichmachern und Gleitmitteln behandelt, um ihre Verarbeitungseigenschaften zu verbessern, was jedoch die acidifizierenden Eigenschaften des Fasermaterials vermindert.

Ein weiteres Verfahren, bei dem ein bekanntes Material, z.B. modifizierte Viskosefaser, mit einer Substanz versetzt wird, die bei Gebrauch zu einer Absenkung des pH-Wertes führt, wird in der DE-OS 27 09 132 beschrieben. Zellstoff-Fasern werden mit Monochloressigsäure zu Carboxymethylcellulose umgesetzt; die resultierenden Carboxymethylcellulose-Fasern weisen einen durchschnittlichen Substitutionsgrad von 0,4 bis 2,0 auf. Diese Veretherung wird an der "fertigen" Faser durchgeführt. Nachteilig ist, daß diese nachträgliche Veretherung zu für die genannten Zwecke nur schlecht geeigneten Fasern mit schleimiger Oberfläche führt. Außerdem enthalten aus derartigen Fasern hergestellte Materialien freie Carboxylgruppen nur an der Faseroberfläche, was dazu führt, daß die in den Körperflüssigkeiten vorliegenden kationischen Substanzen eine schnelle und erschöpfende Desaktivierung der Faseroberfläche herbeiführen.

Die vorliegende Erfindung betrifft nun die Verwendung von den pH-Wert regulierenden Materialien aus chemisch modifizierter Viskosefaser mit einem Grad der Substitution durch Carboxygruppen von 0,01 bis 0,3 und einem Faser-pH-Wert von 3,0 bis 4,0 für Hygienemittel.

Als Fasern für die erfindungsgemäß zu verwendenden,

Patentanmeldung D6861EP

den pH-Wert regulierenden Materialien sind z.B. VISCOSORB$^R$-Fasern ( Hersteller: Chemiefaser Lenzing AG) einsetzbar. Es sind jedoch auch andere Fasern geeignet, die die genannten Anforderungen erfüllen. Als geeignete Fasern werden Fasern aus solchen Polymeren verstanden, die vor der Herstellung der eigentlichen Faser an den Hydroxygruppen des Rohmaterials bis zu einem Substitutionsgrad zwischen 0,01 und 0,3 auf an sich bekanntem Wege verethert worden sind. Das führt dazu, daß die Carboxylgruppen nicht nur auf der Faseroberfläche liegen, sondern über den gesamten Faserquerschnitt verteilt sind.

Die genannten Fasern, die für pH-Wert-regulierende Materialien geeignet sind, haben einen Carboxylgruppengehalt von 1,9 bis 2,7% und besitzen dadurch saure Eigenschaften. Der Faser-pH-Wert, bestimmt nach dem Extrapolationsverfahren DIN 54 275, liegt zwischen 3,0 und 4,0, bevorzugt zwischen 3,4 und 3,9. Je nach Herstellungsbedingungen ist dieser Faser-pH-Wert jedoch variabel.

Die beschriebenen Fasern lassen sich nach üblicher Verfahrensweise zu Hygienemitteln, insbesondere Menstruationstampons, Binden und Slipeinlagen sowie Kosmetikwatte verarbeiten. Sie weisen im Vergleich zu herkömmlicher, stark saugfähiger Watte eine Reihe von Vorteilen auf.

Infolge des niedrigen Substitutionsgrades nach Überführung der Carboxylatgruppe in die freie Säureform entspricht die Saugkapazität und Saugkraft der Materialien derjenigen von normaler nativer bzw. regenerierter Cellulose oder Baumwolle. Vaginale Schädigungen durch Austrocknungseffekte infolge überhöhter

Patentanmeldung D6861 EP

0138178

HENKEL KGaA
ZR-FE/Patente

Saugfähigkeit wurden nicht beobachtet. Die kolposkopischen Untersuchungen von Frauen vor und nach der Verwendung von Tampons, für die die pH-Wert regulierenden Materialien verwendet werden, ließen keine Schädigung bzw. negative Auswirkungen erkennen.

Da die Modifikation, d.h. die Veretherung bereits am Zellulose-Rohmaterial vor der Faserherstellung durchgeführt wird und deshalb die Carboxylgruppen über den gesamten Faserquerschnitt verteilt vorliegen, haben die pH-Wert-regulierenden Materialien eine deutlich bessere Pufferkapazität als erst an der fertigen Faser modifizierte Materialien. So stellt sich bei der Verwendung der Materialien in Menstruationstampons bei der Anwendung ein azider pH-Wert ein und wird aufrechterhalten, der mit Werten um 5,0 den physiologischen Bedingungen Rechnung trägt. Im Gegensatz dazu ist der pH-Bereich bei der Verwendung üblicher Tampons im unphysiologischen neutralen bis alkalischen pH-Bereich fixiert.

Die Messung der vaginalen pH-Werte nach Gebrauch von Tampons, für die erfindungsgemäß azide Materialien verwendet werden, liegt im Bereich von 4,0 bis 5,0, während er beim Gebrauch üblicher Tampons aus Materialien nach dem Stand der Technik im Bereich von 6,0 bis 7,0 gemessen wurde. Durch Erhöhung des pH-Wertes über die physiologische Grenze von 4,5 hinaus wird jedoch für die Vaginalflora atypisches Bakterienwachstum und Pilzbefall gefördert. Bei Disposition zu vaginalen Infekten flackern diese insbesondere nach der Menstruation infolge des erhöhten pH-Wertes besonders leicht wieder auf. Durch die Verwendung von Tampons, für deren Herstellung pH-Wert regulierende Materialien verwendet werden, wird diese

Patentanmeldung    D 6861 EP

Situation deutlich verbessert.

Zudem werden durch eine Erhöhung des pH-Wertes über 5 die Bedingungen für das Wachstum des milchsäure-produzierenden und für ein gesundes Vaginalmilieu wichtigen Lactobacillus acidophilus beeinträchtigt. Eine Erniedrigung des Wertes bei oder nach Verwendung von erfindungsgemäßen Tampons schafft somit günstige Ausgangsvoraussetzungen für ein gesundes Vaginalmilieu.

Zudem konnte gezeigt werden, daß die Zersetzung von exkretierten Körperflüssigkeiten, wie z.B. Menstrualblut, durch Bakterien und die damit verbundene Geruchsentwicklung durch Verwendung der pH-Wert regulierenden Materialien im Vergleich zu herkömmlichen Materialien stark verzögert ist. Der Grund liegt darin, daß das Wachstum der für die Zersetzung verantwortlichen Bakterien durch die physiologischen, leicht sauren pH-Werte inhibiert wird. Man kann die genannten Materialien auch für andere Zwecke, beispielsweise zur Herstellung von Menstruationsbinden und Slipeinlagen verwenden.

Die Erfindung wird durch die nachstehenden Beispiele näher erläutert.Die Herstellung der untersuchten Watten für die den pH-Wert-regulierenden Materialien erfolgte dabei durch Überführung der Carboxylatgruppen von aus carboxyliertem Zellstoff über das Viskoseverfahren hergestellter, in alkalisierter Form vorliegender Carboxyalkylcellulose, z.B. der auf dem Markt erhältlichen Produkte VISCOSORB 1S$^R$ und VISCOSORB 1N$^R$, in die freie Säureform mit 0,2%iger wäßriger Salzsäure, wie oben beschrieben.

Patentanmeldung    D6861 EP                    HENKEL KGaA
                                               ZR-FE/Patente

0138178

## Beispiel 1

Herstellung von Tampons aus den aciden Materialien

Die Materialien wurden, wie zur Tamponherstellung üblich, an einer Krempel aufgelöst und zu einem Watteband mit einer auf die Fläche bezogenen Masse von ca. $630 g/m^2$ verarbeitet. Die Verfestigung erfolgte durch Vernadelung.

Auf Wattebandabschnitte (40 x 90mm) wurde die Rückholkordel in Längsrichtung aufgenäht. Die Verpressung erfolgte nach Konditionierung im Klimaschrank (20°C, 65% rel. Luftfeuchte) axial und radial nach dem Tampax-Verfahren. Alle geprüften Watten ließen sich problemlos verarbeiten und führten zu sehr guten Preßkörpern.

Die aziden Watten zeigten eine bessere Kompressibilität als herkömmliche Watten, so daß unter gleichen Herstellbedingungen Tampons von etwas höherer Dichte erhalten wurden.

Mit diesen Materialien können Tampons und auch andere Hygienemittel auch nach anderen Verfahren hergestellt werden.

## Beispiel 2

Bestimmung der Saugkapazität von Tampons aus pH-Wert-regulierenden Materialien

Der Test zur Bestimmung der Saugkapazität wurde in einer künstlichen Vagina (Syngina) durchgeführt. Dieses Gerät besteht aus einem Plastikrohr

Patentanmeldung    D6861 EP

(Innendurchmesser 35mm), in dem eine elastische Folie gespannt ist. Nach Einschieben des Tampons wird zwischen Rohrwand und Folie ein definierter Druck, z.B. 30 oder 20mbar, mit Preßluft aufgebaut. Dabei umschließt die Folie eng den Tampon.

Mit einer Tropfgeschwindigkeit von ca. 3,5 bis 4,0ml / min wird dann die gefärbte Testflüssigkeit aus einer Meßbürette bis zum Durchdringen des ersten Tropfens am unteren Ende des Tampons aufgegeben. Die durch den Tampon aufgenommene Flüssigkeitsmenge wird an der Bürette abgelesen. Die Messung kann entweder isobar oder mit steigendem Druck (Aufquellen des Tampons) erfolgen. Die Prüfbedingungen, die geometrischen Parameter der Tampons sowie die Ergebnisse sind in der nachfolgenden Tabelle angegeben.

Zur Berechnung der spezifischen Saugleistung in ml/g Watte wurden die geometrischen Parameter der Tampons sowie ihr Gewicht bestimmt.

Tabelle

Geometrische Parameter und Saugkapazität von Tampons

| I) Test-flüssigkeit Wasser | Danufil$^R$-Höchst | VISCOSORB 1N$^R$ (Säure-modif) | VISCOSORB 1S$^R$ (Säure-modif.) |
|---|---|---|---|
| Watte-gewicht (g) | 2,34 | 2,35 | 2,35 |
| Länge (mm) | 38,24 | 35,78 | 36,46 |
| Durch-messer (mm) | 12,11 | 12,13 | 12,10 |

Patentanmeldung    D6861 EP

HENKEL KGaA
ZR-FE/Patente

Fortsetzung der Tabelle

Geometrische Parameter und Saugkapazität von Tampons

| I) Test-flüssigkeit Wasser | Danufil[R]-Höchst | VISCOSORB 1N[R] (Säure-modif) | VISCOSORB 1S[R] (Säure-modif.) |
|---|---|---|---|
| Volumen (ml) | 4,41 | 4,14 | 4,19 |
| Dichte (g/ml) | 0,531 | 0,568 | 0,561 |
| Aufnahme (ml/Tampon) | 7,22 | 6,80 | 6,76 |
| Aufnahme (ml/g Watte) | 3,09 | 2,89 | 2,88 |

| II) Test-flüssigkeit Serum | Danufil[R]-Höchst | VISCOSORB 1N[R] (Säure-modif.) | VISCOSORB 1S[R] (Säure-modif.) |
|---|---|---|---|
| Wattege-wicht (g) | 2,33 | 2,33 | 2,33 |
| Länge (mm) | 38,08 | 34,98 | 35,98 |
| Durchmesser (mm) | 12,09 | 12,15 | 12,06 |
| Volumen (ml) | 4,38 | 4,07 | 4,11 |
| Dichte (g/ml) | 0,532 | 0,572 | 0,567 |
| Aufnahme (ml/Tampon) | 7,76 | 7,46 | 7,46 |
| Aufnahme (ml/g Watte) | 3,33 | 3,20 | 3,20 |

Patentanmeldung D6861 EP

HENKEL KGaA
ZR-FE/Patente

Ergebnis:

Die Saugfähigkeit der Tampons aus aziden Watten stimmt im Wesentlichen mit der von Tampons aus üblicher Watte überein. Die geringfügige Erniedrigung bei den Tampons aus den aciden Materialien resultiert aus der etwas höheren Dichte dieser Tampons, da die Kompressibilität der Materialien unter gleichen Herstellbedingungen etwas besser ist als die herkömmlicher Watte.

Beispiel 3

Einfluß von azider Watte auf die Zersetzung von Menstrualblut und in Folge auftretende Geruchsbildung

Menstrualblut (3-17 ml) wurde von 11 verschiedenen Probandinnen aus der Scheide gesammelt.

Jeweils 250 mg Watte wurden in Reagenzröhrchen mit 1,0 ml Menstrualblut beaufschlagt und bei 37°C inkubiert. Nach verschiedenen Zeiten (0-8h) erfolgte durch 5 Personen eine geruchliche Beurteilung.

Ergebnis:

Die aziden Watten aus VISCOSORB 1N$^R$ und VISCOSORB 1S$^R$ zeigten im Vergleich zu herkömmlicher Watte eine deutliche Inhibierung der Geruchsbildung, die bei der 1S-Form stärker ausgeprägt war als bei der 1N-Form.

Beispiel 4

Verträglichkeit im in-vivo-Test

Je 10 Tampons wurden von 25 Frauen während einer Men-

struationsperiode benutzt, wobei die Tragedauer bis zu 12h betrug. Die Frauen wurden vor Beginn der Anwendung und unmittelbar danach kolposkopisch untersucht, wobei insbesondere auf Hautreizungen der Scheide geachtet wurde. In keinem Fall konnte ein negativer Einfluß der Tampons festgestellt werden. Auch die subjektive Beurteilung der Tampons durch die Probandinnen war positiv. Nebeneffekte wurden nicht festgestellt.

**Beispiel 5**

Messung des vaginalen pH-Wertes nach Anwendung der Tampons aus den pH-Wert regulierenden Materialien

Der vaginale pH-Wert wurde mit einer Vaginalelektrode (Dr. W. Ingold KG) vor und nach Anwendung von Tampons aus aciden und normalen Watten gemessen. Im Mittel wurde eine Absenkung der Werte um ca. 1 - 2 Einheiten, nämlich von pH-Werten zwischen 6,0 und 7,0 nach Verwendung üblicher Tampons auf physiologische Werte von 4,0 bis 5,0 nach Verwendung von Tampons aus azider Watte, beobachtet.

**Beispiel 6**

pH-Wert von Tampons nach Aufnahme von Menstrualblut

Der mittlere pH-Wert der Tamponwatte betrug bei einer Menge von ca. 1ml Menstrualblut/0,25g Watte bei acidifiziertem VISCOSORB 1S$^R$ 5,0, bei acidifiziertem VISCOSORB 1N$^R$ 5,5, im Vergleich dazu bei Normalwatte 7,1. Gemessen wurden Werte an Tampons von 8 verschiedenen Probandinnen.

Patentanmeldung    D6861 EP

HENKEL KGaA
ZR-FE/Patente

Dies bedeutet, daß der Tampon-pH-Wert nach Aufnahme von Menstrualblut bei azider Watte gemäß um ca. 2 Einheiten niedriger liegt als der von herkömmlicher Watte.

Patentanmeldung

D6861EP

HENKEL KGaA
ZR-FE/Patente

0138178

Patentanspruch

1. Verwendung von den pH-Wert regulierenden Materialien, die aus einheitlicher, chemisch modifizierter Viskosefaser mit einem Grad der Substitution durch Carboxygruppen von 0,01 - 0,3 und einem Faser-pH-Wert von 3,0 bis 4,0 bestehen, für Hygienemittel, insbesondere für Menstruationstampons, Binden und Slipeinlagen.